(19) Europäisches Patentamt
European Patent Office
Office européen des brevets

(11) Publication number: **0 288 773 B1**

# EUROPEAN PATENT SPECIFICATION

(45) Date of publication of patent specification: **08.01.92**

(51) Int. Cl.⁵: **A23L 1/28**, A23L 1/23, C12P 7/04

(21) Application number: **88105223.7**

(22) Date of filing: **31.03.88**

(54) **Mushroom flavour.**

(30) Priority: **01.05.87 US 45608**

(43) Date of publication of application:
**02.11.88 Bulletin 88/44**

(45) Publication of the grant of the patent:
**08.01.92 Bulletin 92/02**

(84) Designated Contracting States:
**AT BE CH DE FR GB IT LI LU NL SE**

(56) References cited:

ZEITSCHRIFT FÜR LEBENSMITTEL - UNTER-SUCHUNG UND - FORSCHUNG, vol. 175, 1982, pages 186-190; M. WURZENBERGER et al.: "The enzymic oxidative breakdown of linoleic acid in mushrooms (Psalliota bispora)

IDEM

RIVISTA ITALIANA ESSENZE, PROFUMI, PIANTE, OFFICINALI, AROMI, SAPONI, COS-METICI, 1977, pages 182-185; P. VAROQUAUX et al.: "Formation enzimatique de l'arôme de champignon de couche (Agaricus bisporus linnaeus)"

IDEM

JOURNAL OF AGRICULTURAL AND FOOD CHEMISTRY, vol. 30, 1982, pages 89-93, American Chemical Society; R. TRESSL et al.: "Formation of eight-carbon and ten-carbon components in mushrooms (Agaricus campestris)"

(73) Proprietor: **SOCIETE DES PRODUITS NESTLE S.A.**
Case postale 353
CH-1800 Vevey(CH)

(72) Inventor: **Kibler, Lawrence Allan**
23, Glen Brook Drive
New Milford, Conn. 06776(US)
Inventor: **Kratky, Zdenek**
110 Candlewood Lake Road
New Milford, Conn. 06776(US)
Inventor: **Tandy, John Stewart**
207a Blue Swamp Road
Litchfield, Conn. 06759(US)

## Description

The present invention relates to a mushroom flavour and more particularly to the preparation of a natural mushroom flavourant which can impart fresh and cooked mushroom flavours to foodstuffs.

The characteristic and major volatile flavouring component of many mushroom species is 1-octen-3-ol, known as "mushroom alcohol". B.O. de Lumen et al, Journal of Food Science 43:698, 1978 demonstrated a lipoxygenase system in mushrooms catalysing the conversion of linoleic acid into 1-octen-3-ol. R. Tressl et al, Journal of Agricultural Food Chemistry 30:89, 1981 described an increase of 1-octen-3-ol content in mushrooms homogenised with linoleic acid. Though this document refers to the use of potassium linoleate, proper analysis of the text reveals that this compound was only used as the starting material. Since the ph of the aqueous system obtained by addition of phosphate buffer is 6,8 linoleate acid must be present (in the beginning of the text (page 89, left hand column, line 27) the author speaks therefore of linoleic acids). Wurzenberger and Grosh, Biochim. Biophys. Acta. (795:163, 1984 and 794:25, 1984) described an oxidative cleavage of linoleic acid into 1-octen-3-ol catalysed by protein fraction prepared from extract of mushrooms, Psalliota bispora. In all the above described cases, pure linoleic acid was used but the increase in the 1-octen-3-ol content did not exceed two-fold.

We have now found, surprisingly, that when a watersoluble salt of linoleic acid is used as the precursor instead of linoleic acid, the increase in the 1-octen-3-ol content can be more than quadrupled.

Accordingly, the present invention provides a process for producing a mushroom flavourant which comprises homogenising mushrooms and, during or after homogenisation, contacting the mushrooms with an aqueous medium containing a water-soluble salt of linoleic acid and oxygen.

The process of this invention does not exclude the possibility of contacting the mushrooms with oxygen before the salt of linoleic acid although there is no advantage in doing so.

The homogenisation of the mushrooms may be carried out as, for example, in a batch process, during the period of contact with the aqueous medium containing oxygen and the salt of linoleic acid and in this case the time of contact may be from about 1 to about 20 minutes, preferably from 2.5 to 15 minutes and especially from 5 to 12.5 minutes. Alternatively, the mushrooms may be homogenised before contact with the oxygen as, for example, in a continuous process where the mushrooms are first homogenised in the presence of the aqueous medium containing the salt of linoleic acid in a few seconds, for example, from about 1 to about 30 seconds and then contacted with the oxygen with intensive mixing for a period of time from a few seconds to several minutes, for instance from about 1 to about 10 minutes and preferably from 2.5 to 7.5 minutes. After homogenisation, the mushrooms may conveniently be transferred to a separate reaction vessel for contact with the oxygen. Preferably the time span between homogenisation and contact with the oxygen is as short as possible.

The water-soluble salt of linoleic acid is conveniently the sodium or the potassium salt. Preferably, the water-soluble salt of linoleic acid is obtained by the chemical or enzymatic hydrolysis of a vegetable oil in which linoleic acid occurs as glyceride and then alkalising, for example, with NaOH or KOH. Suitable vegetable oils are cottonseed, soybean, peanut, corn, sunflower seed, poppy seed, linseed and perilla oils but safflower oil is especially advantageous. The hydrolysis of the vegetable oil is conveniently carried out enzymatically, for instance, using lipase obtained from the pancreas or of microbiological origin.

The amount of the water-soluble salt of the linoleic acid used may be from about 0.1 to about 5 parts per 100 parts, preferably from 0.2 to 2.5 parts and especially from 0.25 to 1 part per 100 parts by weight of mushrooms.

The mushroom source may be any of the commercially available cultivar of the Agaricaceae Family such as Agaricus bisporus, Agaricus bitorquis or Agaricus campestris. Brown strains are slightly preferred. While it is advantageous to use the mushrooms soon after harvesting, mushrooms which are refrigerated may also be used because they retain their capacity to convert linoleic acid into 1-octen-3-ol. Conveniently, the mushrooms are washed before use, for example, by means of a belt washer where the mushrooms are sprayed with water, optionally in the presence of sodium bisulphite. Immersion of the mushrooms in water for prolonged periods of time should be avoided as the anaerobic conditions created under such circumstances cause the mushrooms to quickly metabolise their own 1-octen-3-ol and their capacity to convert linoleic acid to 1-octen-3- ol in subsequent homogenisation is partially lost.

The weight ratio of the mushrooms to the aqueous medium may vary from 1:0.01 to 1:10, preferably 1:0.1 to 1:5, more preferably from 1:0.5 to 1:2.5 and especially from 1:1 to 1:2.

The homogenisation of the aqueous medium containing mushrooms and the water-soluble salt of linoleic acid may be carried out batchwise or continuously and the duration of the homogenisation may vary from a few seconds to several minutes provided that there is intimate contact of the linoleic acid salt and oxygen with the mushroom homogenate. Preferably, forced aeration is used to achieve the intimate contact of oxygen with the homogenate and the amount of air passing through the mushroom homogenate may be, for example, from 1 to 20 m³/min and preferably from 2 to 10 m³/min per 600 kg of homogenate. A mixture of oxygen and inert gases e.g. carbon dioxide, nitrogen, could also be used instead of air.

The homogenisation is preferably carried out at a temperature below 30°C, preferably from 12°C to 28°C and especially from about 15°C to about 25°C. The pH during the homogenisation is advantageously from 5.5 to 8.0.

Preferably, additives such as flavouring plant extracts, edible oils and carriers suitable for spray drying are added to the homogenate. An especially preferred flavouring plant extract is St. John's Bread commonly known as locust bean extract or carob bean extract which is a concentrated extract prepared from the fruit of Ceratonia siliqua, a tree native to the Mediterranean area (carob tree) and provides unexpectedly desirable sweetness and a base note. The amount of flavouring plant extract added may be from about 0.1 to about 10%, preferably from 0.5 to 5% and especially from 1 to 4% by weight based on the weight of mushrooms. The edible oil may be of vegetable or animal origin and a suitable edible oil is partially hydrogenated cotton seed and soya oil, and may conveniently be added in amounts ranging from about 0.1 to about 15%, preferably from 0.2 to 10% and especially from 0.5 to 6% by weight based on the weight of mushrooms. The amount of carrier used may be from about 10% to about 200% and preferably from 20% to 100% by weight based on the weight of the mushrooms. Examples of suitable carriers are low DE maltodextrin, high DE maltodextrins, modified starches or gums such as gum arabic.

After mixing the additives, the slurry is conveniently pasteurised, homogenised and spray dried.

The following Examples further illustrates the present invention.

Example 1

A) Gum arabic (1.5 kg), sodium chloride (0.75 kg) and calcium chloride (0.37 kg) are dissolved in 150 kg of water. 15 kg safflower seed oil (high linoleic acid type), are added and the mixture is emulsified by means of a high energy mixer. The pH of the emulsion is maintained at 8 and the temperature at 40°C. Pancreatic lipase (0.37 kg, 92500 lipase unit) is added to the emulsion which is stirred and the pH kept at around 8 by the addition of NaOH solution. The endpoint of the reaction is indicated by no further changes in the pH of the mixture. HCl is then added to bring the pH to 2.5. The mixture is allowed to stand to allow phase separation. The upper phase, containing fatty acids, is separated, 30 kg of water is added and the pH is adjusted to 9.4 by the addition of NaOH to give a solution containing approximately 10 kg of sodium linoleate.

B) The amount of this solution which contains 2 kg of sodium linoleate (i.e. one fifth of the total) is added to 400 litres of water at 20°C in a 1000 litre high energy liquefier equipped with an air sparger and the mixture is well dispersed. 400 kg of washed mushrooms (Agaricus bisporus) are added and the mixture is homogenised for 10 minutes at high speed with air introduced through the air sparger. The homogenate is then mixed with 16.4 kg of vegetable oil, 166.4 kg of modified starch (a convenient carrier for spray-drying) and 166.4 kg of low DE maltodextrin. After mixing this slurry, it is homogenised at 2000 psi, pasteurised and spray-dried to give an attractive mushroom flavour containing on a dry weight basis 10.3% mushrooms, 84% carriers, 4% oils and 3% moisture. The concentration of 1-octen-3-ol is 1000 ppm.

Example 2

A similar procedure to that described in Example 1 is followed except that after homogenisation of the mushrooms with the sodium linoleate, the homogenate is mixed with 10.0 kg of solid extract of St. John's Bread, in addition to the vegetable oil, modified starch and low DE maltodextrin. After mixing this slurry, homogenising, pasteurising and spray drying as in Example 1 the flavour obtained has an exceptionally desirable overall sweet mushroom flavour and significant increase in the background note when compared with the flavour in Example 1 owing to the amazingly high and synergistic effect of the St. John's Bread. The flavourant contains on a dry weight basis 10% mushrooms, 80% carriers, 4% oils, 2.2% plant extracts and 3% moisture.

Comparative Example A

A similar procedure to that described in Example 1B is followed except that, instead of the solution containing 2 kg of sodium linoleate, the same amount of an aqueous emulsion containing 2 kg of linoleic acid is used. The concentration of the 1-octen-3-ol in the spray-dried flavour is only 400 ppm.

Claims

1. A process for producing a mushroom flavourant which comprises homogenising mushrooms and, during or after homogenisation, contacting the mushrooms with an aqueous medium containing a water-soluble salt of linoleic acid and oxygen.

2. A process according to claim 1 wherein mushrooms are homogenised first in the presence of the salt of linoleic acid in the aqueous medium and then the homogenised mushrooms are contacted with the oxygen.

3. A process according to claim 1 wherein mushrooms are homogenised in the presence of the salt of linoleic acid in the aqueous medium while simultaneously introducing oxygen into the medium.

4. A process according to claim 2 wherein the salt of linoleic acid is selected from the group consisting of a sodium and a potassium salt of linoleic acid and wherein the salt is in an amount of from about 0.1 to about 5 parts per 100 parts by weight of the mushrooms homogenised and wherein the mushrooms are homogenised for from about 1 second to about 30 seconds and then contacted with the oxygen for from about 1 minute to about 10 minutes at a temperature of from about 12°C to about 28°C.

5. A process according to claim 3 wherein the water soluble salt of linoleic acid is selected from the group consisting of a sodium and a potassium salt of linoleic acid and wherein the salt is in an amount of from 0.1 to 5 parts per 100 parts by weight of the mushrooms homogenised and wherein the mushrooms are homogenised and the oxygen is introduced for from about 1 minute to about 20 minutes at a temperature of from about 15°C to about 25°C.

6. A process according to claim 1 wherein air provides the oxygen and is introduced by forced aeration into the aqueous medium.

7. A process according to claim 1 or 2 or 3 further comprising adding at least one of a flavouring plant extract additive, of an edible oil additive and of a carrier additive suitable for spray drying the homogenate.

8. A process according to claim 7 wherein the flavouring plant extract additive is added in an amount from about 0.1% to about 10% by weight based upon the weight of the mushrooms which were homogenised, the edible oil additive is added in an amount of from about 0.1% to about 15% by weight based upon the weight of the mushrooms which were homogenised and the carrier additive is added in an amount of from about 10% to about 200% by weight based upon the weight of the mushrooms which were homogenised.

9. A process according to claim 7 wherein the flavouring plant extract additive is locust bean extract.

10. A process according to claim 1 or 2 or 3 further comprising spray-drying the homogenate.

11. A process according to claim 7 further comprising spray drying the homogenate and additives.

12. A process according to claim 9 further comprising spray-drying the homogenate and additives.

13. The product of the process of claim 1 or 2 or 3.

14. The product of the process of claim 9.

15. The product of the process of claim 12.

**Revendications**

1. Procédé de production d'un agent aromatisant à base de champignons, qui consiste à homogénéiser des champignons et, pendant ou après l'homogénéisation, à mettre en contact les champignons avec un milieu aqueux contenant un sel hydrosoluble d'acide linoléique et de l'oxygène.

2. Procédé suivant la revendication 1, dans lequel les champignons sont homogénéisés tout d'abord en présence du sel d'acide linoléique dans le milieu aqueux, puis les champignons homogénéisés sont mis en contact avec l'oxygène.

3. Procédé suivant la revendication 1, dans lequel les champignons sont homogénéisés en présence du sel d'acide linoléique dans le milieu aqueux, avec introduction simultanée d'oxygène dans le milieu.

4. Procédé suivant la revendication 2, dans lequel le sel d'acide linoléique est choisi dans le groupe comprenant un sel de sodium et un sel de potassium d'acide linoléique et dans lequel le sel est présent en une quantité d'environ 0,1 à environ 5 parties pour 100 parties en poids des champignons homogénéisés, et les champignons sont homogénéisés pendant un temps d'environ 1 seconde à environ 30 secondes, puis sont mis en contact avec l'oxygène pendant un temps d'environ 1 minute à environ 10 minutes à une température d'environ 12°C à environ 28°C.

5. Procédé suivant la revendication 3, dans lequel le sel hydrosoluble d'acide linoléique est choisi dans le groupe comprenant un sel de sodium et un sel de potassium d'acide linoléique, le sel est présent en une quantité de 0,1 à 5 parties pour 100 parties en poids des champignons homogénéisés, et les champignons sont homogénéisés et l'oxygène est introduit pendant un temps d'environ 1 minute à environ 20 minutes à une température d'environ 15°C à environ 25°C.

6. Procédé suivant la revendication 1, dans lequel l'air fournit l'oxygène et est introduit par aération forcée dans le milieu aqueux.

7. Procédé suivant la revendication 1, 2 ou 3, consistant en outre à ajouter au moins un additif consistant en un extrait végétal aromatisant, au moins un additif consistant en une huile comestible et au moins un additif servant de support, convenant pour le séchage de l'homogénat par pulvérisation.

8. Procédé suivant la revendication 7, dans lequel l'additif consistant en un extrait végétal aromatisant est ajouté en une quantité d'environ 0,1% à environ 10% en poids, sur la base du poids des champignons qui ont été homogénéisés, l'additif consistant en une huile comestible est ajouté en une quantité d'environ 0,1% à environ 15% en poids sur la base du poids des champignons qui ont été homogénéisés, et l'additif servant de support est ajouté en une quantité d'environ 10% à environ 200% en poids sur la base du poids des champignons qui ont été homogénéisés.

9. Procédé suivant la revendication 7, dans lequel l'additif consistant en un extrait végétal aromatisant est l'extrait de caroube.

10. Procédé suivant la revendication 1, 2 ou 3, consistant en outre à sécher l'homogénat par pulvérisation.

11. Procédé suivant la revendication 7, consistant en outre à sécher l'homogénat et les additifs par pulvérisation.

12. Procédé suivant la revendication 9, consistant en outre à sécher l'homogénat et les additifs par pulvérisation.

13. Produit obtenu par la mise en oeuvre du procédé suivant la revendication 1, 2 ou 3.

14. Produit obtenu par la mise en oeuvre du procédé suivant la revendication 9.

15. Produit obtenu par la mise en oeuvre du procédé suivant la revendication 12.

**Patentansprüche**

1. Verfahren zur Herstellung eines Pilzaromas, welches ein Homogenisieren von Pilzen und, während oder nach dem Homogenisieren, ein In-Kontakt-Bringen der Pilze mit einem wäßrigen, ein wasserlösliches Salz von Linolsäure enthaltenden Medium und mit Sauerstoff umfaßt.

2. Verfahren nach Anspruch 1, worin die Pilze zunächst in Anwesenheit des Salzes der Linolsäure in dem wäßrigen Medium homogenisiert werden und hierauf die homogenisierten Pilze mit dem Sauerstoff in Kontakt gebracht werden.

3. Verfahren nach Anspruch 1, worin die Pilze in Gegenwart des Salzes der Linolsäure im wäßrigen Medium homogenisiert werden, während gleichzeitig Sauerstoff in das Medium eingeführt wird.

4. Verfahren nach Anspruch 2, worin das Salz der Linolsäure aus der aus einem Natrium- und einem Kaliumsalz von Linolsäure bestehenden Gruppe ausgewählt wird und worin das Salz in einer Menge von etwa 0,1 bis etwa 5 Teilen je 100 Gewichtsteilen der homogenisierten Pilze vorliegt und worin die Pilze etwa 1 bis etwa 30 Sekunden lang homogenisiert werden und hierauf etwa eine Minute bis etwa 10 Minuten lang bei einer Temperatur von etwa 12°C bis etwa 28°C mit dem Sauerstoff in Kontakt gebracht werden.

5. Verfahren nach Anspruch 3, worin das wasserlösliche Salz der Linolsäure aus der aus einem Natrium- und einem Kaliumsalz der Linolsäure bestehenden Gruppe ausgewählt wird und worin das Salz in einer Menge von 0,1 bis 5 Teilen je 100 Gewichtsteilen der homogenisierten Pilze vorliegt und worin während etwa einer Minute bis etwa 20 Minuten bei einer Temperatur von etwa 15°C bis etwa 25°C die Pilze homogenisiert werden und der Sauerstoff eingeführt wird.

6. Verfahren nach Anspruch 1, worin Luft den Sauerstoff zur Verfügung stellt und diese durch Zwangsbelüftung in das wäßrige Medium eingeführt wird.

7. Verfahren nach Anspruch 1 oder 2 oder 3, das weiterhin ein Zusetzen wenigstens eines Additivs aus einem Pflanzenextraktaroma-Additiv, einem eßbares Öl-Additiv und einem Träger-Additiv, das zum Sprühtrocken des Homogenisats geeignet ist, umfaßt.

8. Verfahren nach Anspruch 7, worin das Pflanzenextraktaroma-Additiv in einer Menge von etwa 0,1% bis etwa 10 Gew.-% bezogen auf das Gewicht der Pilze, die homogenisiert wurden, das eßbare Öl-Additiv in einer Menge von etwa 0,1% bis etwa 15 Gew.-%, bezogen auf das Gewicht der Pilze, die homogenisiert wurden, und das Träger-Additiv in einer Menge von etwa 10% bis etwa 200 Gew.-%, bezogen auf das Gewicht der Pilze, die homogenisiert wurden, zugesetzt werden.

9. Verfahren nach Anspruch 7, worin das Pflanzenextraktaroma-Additiv Johannesbrotextrakt ist.

10. Verfahren nach Anspruch 1 oder 2 oder 3, welches weiterhin ein Sprühtrocknen des Homogenisats umfaßt.

11. Verfahren nach Anspruch 7, welches weiterhin ein Sprühtrocken des Homogenisats und der Additive umfaßt.

12. Verfahren nach Anspruch 9, welches weiterhin ein Sprühtrocknen des Homogenisats und der Additive umfaßt.

13. Das Produkt des Verfahrens nach Anspruch 1 oder 2 oder 3.

14. Das Produkt des Verfahrens nach Anspruch 9.

15. Das Produkt des Verfahrens nach Anspruch 12.